# FASCICULE DE BREVET EUROPEEN

(11) **EP 1 227 714 B1**
(45) Date de publication et mention de la délivrance du brevet: **06.05.2004**
(21) Numéro de dépôt: 00976113.1
(22) Date de dépôt: 07.11.2000
(51) Int. Cl.: A01G 31/00, A01G 31/02

(54) **PROCEDE DE PRODUCTION DE METABOLITES A PARTIR DE VEGETAUX EN CULTURE HORS SOL**
VERFAHREN ZUM ERZEUGEN VON METABOLITEN AUS PFLANZEN IN ERDLOSER KULTUR
METHOD FOR PRODUCING METABOLITES FROM PLANTS CULTIVATED IN SOIL-LESS MEDIUM

(30) Priorité: 08.11.1999 FR 9914204
(43) Date de publication de la demande: 07.08.2002
(73) Titulaire: Institut National Polytechnique de Lorraine (INPL), 54500 Vandoeuvre les Nancy (FR); Institut National de la Recherche Agronomique, 75338 Paris Cedex 07 (FR)
(72) Inventeur: GONTIER, Eric, F-54170 Germiny (FR); CLEMENT, Alain, F-54000 Nancy (FR); BOURGAUD, Frédéric, F-54500 Vandoeuvre Les Nancy (FR); GUCKERT, Armand, F-54600 Villers Les Nancy (FR)
(74) Mandataire: Nuss, Pierre
(86) Numéro de dépôt international: PCT/FR2000/003095
(87) Numéro de publication internationale: WO 2001/033942

(56) Documents cités:
- WO-A-96/34522
- GB-A- 2 009 574
- US-A- 5 244 794
- US-A- 5 310 672

## Description

L'invention concerne un procédé de production de métabolites, en continu ou en semi-continu, à partir de plantes en culture hors sol, sans perte de viabilité desdites plantes, ainsi qu'une installation pour la mise en oeuvre de ce procédé.

Les plantes produisent un grand nombre des molécules que l'homme utilise comme médicaments, colorants, arômes, additifs alimentaires ou pesticides. Ces composés sont souvent typiques d'une famille d'un genre ou d'une espèce végétale donnée. On les classe parmi les métabolites secondaires car ils ne semblent pas toujours indispensables à la survie de la plante non stressée (Bentley 1999, Bourgaud et al 1999, Gontier 1993). Beaucoup de ces molécules d'origine végétale servent ou ont servi de modèle pour la synthèse chimique en vue d'une utilisation commerciale. Parfois, les molécules sont complexes et l'extraction à partir de plantes sauvages, de plantes cultivées en champ ou in vitro restent les meilleures sources d'approvisionnement (Herman 1993).

En particulier, pour les molécules à haute valeur commerciale (prix au kilogramme et volume du marché), les cultures de cellules ou de tissus en bioréacteurs ont été développées (Herman 1993). Dans ce cas, les cultures doivent être axéniques, c'est à dire exemptes de micro-organismes (bactéries et champignons) dont la présence pourrait être dommageable à la croissance du matériel végétal et à la production de métabolites par ce dernier. Le maintien de conditions axéniques est parfois difficile dans le temps et est source de charges financières importantes (Gontier 1993). De plus, l'utilisation de systèmes sophistiqués pour la culture in vitro (fermenteurs) ajoute un coût parfois rédhibitoire, rendant le système économiquement non viable. De manière générale, la production de biomasse in vitro est onéreuse. Pour rentabiliser le système, on cherchera à faire produire le maximum de molécules par cette biomasse sans la détruire et en la réutilisant au maximum comme biocatalyseur (Herman 1993).

La stimulation de la production peut être obtenue par ajout de précurseurs dans le milieu de culture (Boitel et al 1997), par perméabilisation réversible de la biomasse ou par stress physique (élévation de température, changement de pH, blessure...), chimique (ajout de sels CuSO4, NaCl, CaCl2, (NH4)2SO4...ou autres perméabilisants EDTA, DMSO, Tween20, autres tensioactifs ou détergents...) ou biochimique (élicitation...)...(Boitel et al 1995-1997, Weather et al 1991, Herman 1993, Mukundan et al 1998)

Le but de telles manipulations est de forcer la voie de biosynthèse en augmentant le flux global des précurseurs vers la molécule recherchée. De nombreuses publications font état de résultats extrêmement positifs obtenus par ces différentes voies (dont : Boitel et al 1995-1997, Weather et al 1991, Herman 1993). Néanmoins, le problème du caractère axénique des cultures reste entièrement posé.

D'autres auteurs, décrivent l'utilisation de cocktails antifongiques et antibiotiques permettant d'assurer une survie du matériel végétal en condition non axénique ("Plant Preservative Mixture" dénommé couramment PPM ®). Dans ce cas, il est difficile de doser les inhibiteurs microbiens de telle sorte que seuls les bactéries et les champignons soient affectés et ce, sans effet négatif sur la biomasse végétale.

Par opposition à la culture in vitro, la production de molécules à partir de plantes récoltées dans la nature ou cultivées en champs est une alternative intéressante. Cependant, la récolte dans la nature peut conduire à la disparition d'espèces en cas de surexploitation (Gontier 1993). Elle peut aussi entraîner d'importantes variations qualitatives et quantitatives de la production. La culture en champ n'est pas toujours réalisable par manque de connaissance de la plante ou, bien pire, pour cause d'impossible retour sur investissement à moyen terme. C'est souvent le cas pour les ligneux- par exemple l'if (Chen et Chen 1997, Ketchum et al 1999)- qui poussent lentement et dont la croissance s'étale sur des décennies.

Depuis plusieurs années, des cultures dites hors sol (Morard 1995, Toda et al 1999) sous serre ou en extérieur se sont développées et permettent maintenant la culture de plantes maraîchères ou ornementales avec un niveau de rentabilité élevé. Les progrès concernant ces techniques ont été fulgurants depuis environ quinze ans tant au niveau des dispositifs, des solutions nutritives que du contrôle et de l'automatisation de ces systèmes (Morard 1995). Jusqu'à ce jour, aucun essai de production de métabolite secondaire à partir de telles cultures n'a été tenté, ni même proposé, avec en particulier, la récupération des métabolites excrétés naturellement ou non à partir des racines des plantes ou même des parties aériennes. En particulier, l'état de l'art ne montre absolument pas, ni ne suggère, qu'il est possible a priori de récupérer de tels métabolites sans que ceux ci ne soient dégradés par la microflore rencontrée dans les cultures hors sol.

Actuellement. on ne sait donc pas :
- si les plantes, de manière générale, relarguent des métabolites secondaires à partir de leurs racines ou de leurs feuilles de telle sorte que ce phénomène puisse être utilisé pour une application industrielle ;
- s'il est possible de forcer un relargage naturel ou non desdits métabolites secondaires chez les plantes cultivées en particulier en culture hors sol ;
- si un traitement favorisant la synthèse et le relargage desdits métabolites secondaires chez la plante va affecter la survie de ladite plante de telle sorte qu'aucune application industrielle ne puisse être envisagée ;
- si lesdits métabolites sont ou non dégradés de manière importante par la microflore susceptible de se développer autour des plantes ainsi cultivées.

Le problème posé à la présente invention consiste par conséquent à proposer un procédé de production de molécules à partir de plantes ne nécessitant pas de conditions axéniques ou stériles, permettant de produire des quantités importantes, rentable économiquement et n'entraînant pas la destruction, même partielle. des plantes en vue de l'extraction des molécules recherchées.

A cet effet, la présente invention a pour objet un procédé de production de molécules à partir de plantes, caractérisé en ce que lesdites plantes sont cultivées en conditions hors sol et sont alimentées par une solution nutritive et/ou aspergées par un liquide de lessivage, ladite solution et/ou ledit liquide étant ensuite récupéré(e)(s) et traité(e)(s) pour en extraire certaines molécules déterminées qu'elle ou il contient et qui ont été libérées par les racines et/ou les parties aériennes desdites plantes.

Selon une première caractéristique de l'invention, ledit procédé peut consister plus précisément à réaliser les étapes suivantes :
a) initiation de la culture des plantes en conditions hors sol permettant la croissance et le développement desdites plantes et l'obtention d'une biomasse suffisamment importante pour rentabiliser le procédé ;
b) récupération des métabolites libérés par les racines ou les parties aériennes des plantes au moyen d'un liquide mis en contact par percolation ou immersion avec les racines, ou par aspersion avec les feuilles desdites plantes ;
c) collecte de la solution usagée ou chargée sous forme de percolat (pour les racines) ou de lessivat (pour les feuilles) et isolement des molécules recherchées contenues dans cette solution par extraction liquide-liquide, liquide-solide, par recristallisation, par sublimation ou par évaporation de l'eau.

Ledit procédé peut également comprendre une étape supplémentaire de perméabilisation forcée des racines ou des parties aériennes des plantes avec une solution contenant notamment des sels, des tensioactifs, des détergents. des solvants, des éliciteurs d'origine fongique ou bactérienne, des dérivés de l'acide jasmonique ou d'autres produits stimulant les défenses naturelles des plantes, de telle sorte que les flux sortants des racines ou des feuilles vers la solution nutritive ou le liquide de lessivage soient augmentés de manière significative et ce, sans perte totale de la viabilité desdites plantes, afin que ces dernières puissent être réutilisées, directement ou après une phase de rinçage et de remise en conditions de culture permettant au moins le rétablissement d'un bon état physiologique desdites plantes.

Dans ces conditions, le procédé comportera les étapes a à e suivantes :
a) initiation de la culture desdites plantes en conditions hors sol permettant la croissance et le développement desdites plantes et l'obtention d'une biomasse suffisamment importante pour rentabiliser le procédé. L'obtention de cette biomasse importante, notamment au niveau racinaire, peut être favorisée par une transformation génétique via Agrobacterium rhizogenes (Hooykaas et Schilperoort 1992) ;
b) perméabilisation des parties racinaires ou aériennes des plantes au moyen de traitement chimique (sels, tensioactifs, détergents, solvants), physique (élévation de température...) ou biochimique (éliciteur d'origine fongique ou non). Pour les traitements chimiques ou biochimiques, cette perméabilisation peut se faire à partir de la solution nutritive des plantes ou par aspersion des parties aériennes ;
c) récupération des métabolites libérés par les racines et/ou par les parties aériennes des plantes par mise en contact desdites plantes avec un liquide ;
d) récupération de la solution nutritive usagée ou du liquide aspergé chargé (autrement appelée dans la suite : percolat pour la perméabilisation par les racines et lessivat pour la perméabilisation par les parties aériennes) et isolement des molécules recherchées contenues dans cette solution (percolat ou lessivat) par évaporation de l'eau ou extraction liquide-liquide (solution nutritive et solvant insoluble dans l'eau), liquide-solide (solution nutritive et support adsorbant hydrophile, hydrophobe, anionique, cationique ou autre mais présentant une forte affinité pour les métabolites recherchés) ou par recristallisation ;
e) rinçage du système racinaire et/ou des parties aériennes des plantes puis remise en conditions de croissance un temps juste suffisant pour permettre de réaliser à nouveau le traitement.

En variante au déroulement séquentiel des étapes de perméabilisation, de récupération et de remise en condition mentionné ci-dessus, dont le rebouclage sur une pluralité de cycles répétitifs autorise une exploitation efficiente desdites plantes, il peut également être prévu que la perméabilisation forcée soit réalisée de manière permanente, en additionnant une solution perméabilisante à la solution nutritive délivrée de manière continue ou à intervalles réguliers aux plantes.

Dans ce dernier cas, les étapes de remise en conditions des plantes peuvent être totalement supprimées en choisissant des doses de substances perméabilisantes peu importantes et en sélectionnant des substances peu agressives ou stressantes pour les plantes, ou pour le moins être espacées avec des intervalles plus importants.

Suite à l'étape de perméabilisation ou à une phase prolongée de perméabilisation, on réalise un rinçage du système racinaire et/ou des parties aériennes des plantes, puis une remise en conditions de croissance desdites plantes pendant un temps juste suffisant pour permettre de réaliser à nouveau un traitement de perméabilisation forcée, suivi ou accompagné d'une étape de récupération des molécules recherchées.

Il peut être également prévu, de manière avantageuse, de renforcer les capacités de biosynthèse de la plante et son potentiel à relarguer des métabolites dans la solution, par l'intermédiaire d'un traitement physique choisi dans le groupe formé par une élévation de la température, une irradiation lumineuse, supplémentaire ou rallongée, une élévation de la teneur en CO₂ dans l'atmosphère en contact avec ladite plante et les combinaisons de plusieurs de ces traitements physiques.

En vue de rentabiliser davantage le procédé selon l'invention, la production et l'excrétion des métabolites synthétisés par les plantes peuvent être renforcées soit par une néosynthèse de ces molécules suite à l'apport de précurseurs de ces métabolites, ces précurseurs de la voie de biosynthèse desdits métabolites étant apportés à la plante par la solution nutritive au niveau des racines ou par aspersion sur les parties aériennes en mélange avec des tensioactifs pour obtenir une pénétration foliaire, soit par un forçage de l'activité photosynthétique au moyen d'un éclairement artificiel pendant une durée plus longue que celle à laquelle la plante est habituée.

Selon un premier mode de réalisation de l'invention, les métabolites recherchés sont libérés par les plantes au niveau de leurs racines et sont récupérés dans la solution nutritive usagée.

Selon un second mode de réalisation de l'invention, les métabolites recherchés sont obtenus à partir des parties aériennes des plantes par douchage ou aspersion desdites plantes avec une solution ou un liquide adapté(e), contenant préférentiellement des tensioactifs, des agents de surfaces et/ou des détergents, la solution de douchage ou d'aspersion chargée étant récupérée séparément ou en mélange avec la solution nutritive pour une extraction et une purification des molécules recherchées.

De manière préférentielle. les plantes utilisées sont choisies dans le groupe des plantes supérieures ou vascularisées, aptes à synthétiser des métabolites d'intérêt économique en grande quantité et en ce que les molécules recherchées correspondent à des métabolites secondaires produits naturellement par ces plantes.

Les métabolites récupérés et isolés sont choisis dans le groupe formé par les alcaloïdes de types tropaniques et indoliques, les anticancéreux, les taxanes ou dérivés du taxol, les furocoumarines, les terpènes, les glycosides, les phénylpropanoïdes, les saponines et les stéroïdes, et, plus généralement les substances non assimilables ou toxiques pour les micro-organismes éventuellement présents dans l'environnement desdites plantes.

Conformément à une caractéristique supplémentaire de l'invention, les plantes utilisées peuvent être modifiées génétiquement pour leur faire produire plus de métabolites ou pour modifier leur morphologie, en augmentant par exemple leur biomasse racinaire suite à une transformation génétique par Agrobacterium rhizogenes ou tumefaciens.

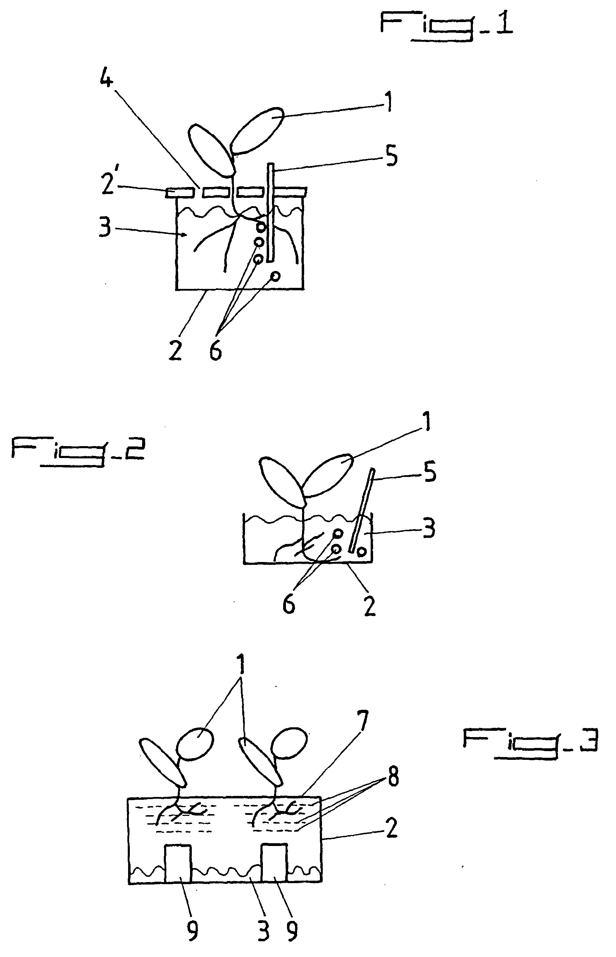

L'invention sera mieux comprise, grâce à la description ci-après, qui se rapporte à des modes de réalisation préférés, donnés à titre d'exemples non limitatifs, et expliqués avec référence aux dessins schématiques annexés, dans lesquels les figures 1 à 8 représentent de manière schématique différentes possibilités de mise en oeuvre du procédé selon l'invention, en relation avec différents moyens et dispositifs matériels.

Ainsi, la figure 1 représente le système utilisé pour l'exemple 1 décrit dans la suite de la présente. Les plantes 1 (Daturas) sont cultivées dans des pots en verre 2 remplis de solution nutritive 3. La tige des plantes 1 est fixée sur le couvercle 2' des pots préalablement percé de trois trous. Un orifice 4 sert à la remise à niveau de la solution nutritive et l'autre permet le passage d'un tuyau d'arrivée d'air 5 qui permet de maintenir une bonne oxygénation de la solution nutritive des plantes par bullage (bulles 6).

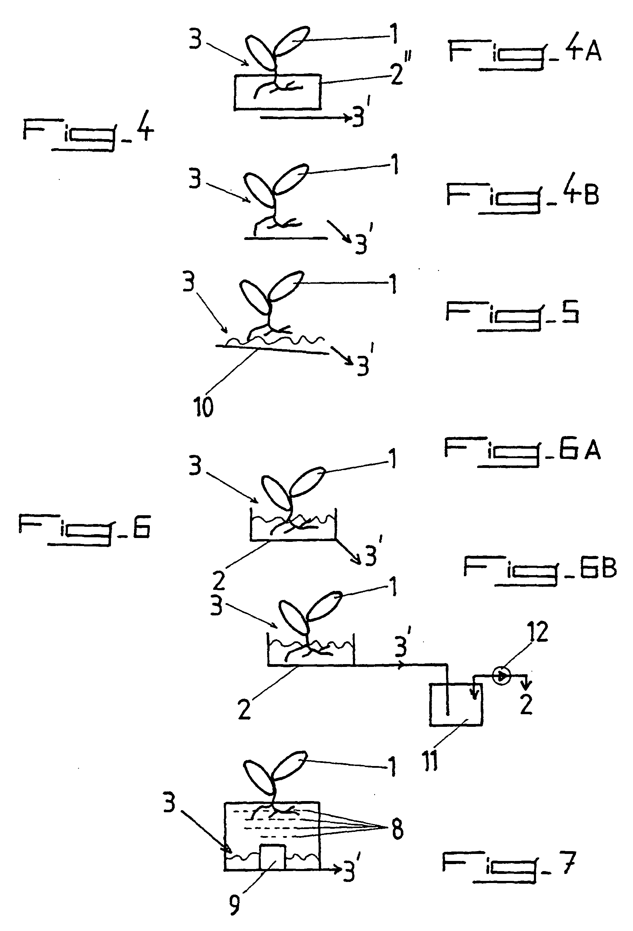

La figure 2 présente le dispositif utilisé pour la culture d'Ifs telle que décrite pour l'exemple 2 dans la suite du texte. Dans ce cas, les plantes 1 sont posées dans le fond d'un bac en 2 PVC (polychlorure de vinyle) rempli de solution nutritive 3, et un bullage d'air assure l'oxygénation de la solution nutritive (bulles 6).

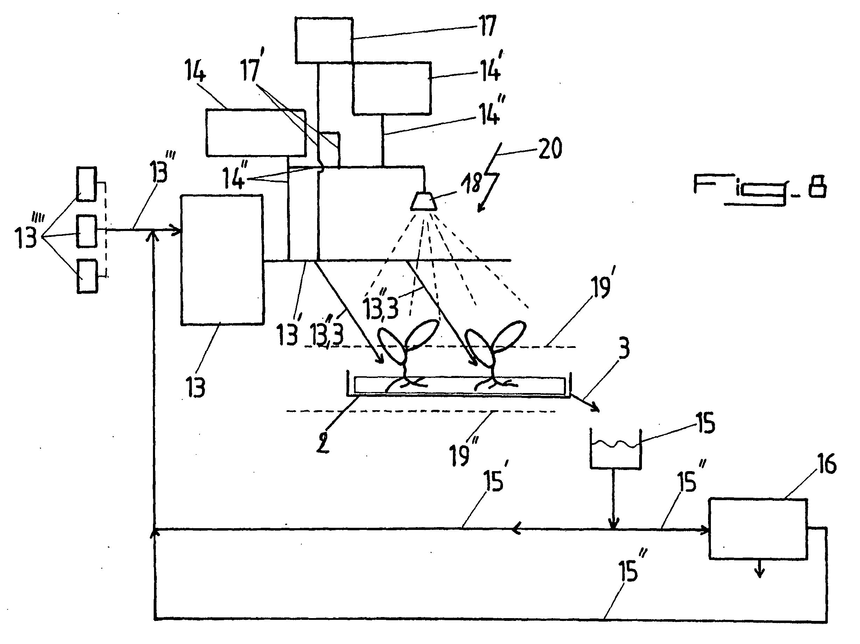

La figure 3 décrit le dispositif utilisé pour la réalisation des travaux décrits dans l'exemple 4 ci-après. Les plantes 1 (Daturas) sont placées sur des plaques percées 7 posées sur un bac de culture 2 en PVC. La solution nutritive est régulièrement pulvérisée sur les racines sous forme de brouillard 8 grâce à des brumisateurs programmables 9.

Les figures 4 à 7 représentent différents dispositifs de culture utilisables pour la production de métabolites à partir de plantes.

Plus particulièrement, les figures 4A et 4B représentent des dispositifs de culture dans lesquels la solution nutritive 3 est apportée aux plantes 1 à l'aide de capillaires. Une fois entrée en contact avec les racines desdites plantes, la solution nutritive usagée 3', aussi appelée percolat, est drainée puis recyclée. Dans ce cas, les plantes peuvent être cultivées avec ou sans substrat 2". Ce substrat peut être de différentes natures telles que: laine de roche, sable, perlite, vermiculite, sol reconstitué, ou de toute autre nature utilisée actuellement en culture hors sol.

Les plantes 1 peuvent aussi être cultivées selon la méthode NFT pour : « Nutrient Film Technique ». Dans ce cas, la solution nutritive 3 est amenée aux racines des plantes 1 par ruissellement (Fig. 5) sur une gouttière ou un plan support 10 légèrement en pente. La récupération de la solution nutritive usagée 3' se fait en bout de gouttière 10 et cette solution peut être recyclée avec ou sans récupération des métabolites qu'elle contient.

Les figures 6A et 6B décrivent des systèmes de culture hors sol avec immersion des systèmes racinaires des plantes 1 dans une solution nutritive. L'apport de solution nutritive 3 et le drainage de la solution usagée 3' peuvent être permanents (système en continu) ou non (système discontinu). L'immersion du système racinaire peut être, elle aussi, permanente (Fig. 6A) avec bullage d'air dans cette même solution nutritive ou temporaire (Fig. 6B). Dans le second cas, le drainage peut se faire vers un bac hermétique 11 en position basse. Le transfert de ce liquide peut alors être fait par une pompe ou par un compresseur 12 qui pousse la solution contenue dans ce bac hermétique 11 vers le bac de culture 2, éventuellement après rajout de substances additionnelles.

La figure 7 décrit un dispositif de culture de type aéroponie, dans lequel les racines des plantes sont régulièrement arrosées par la solution nutritive 3 sous forme de brouillard 8. Ce dernier est obtenu grâce à des brumisateurs 9 placés à l'intérieur du bac de culture 2. La solution nutritive usagée 3' peut être soutirée par le bas.

La figure 8 montre de manière schématique une installation pour la mise en oeuvre, à grande échelle et le cas échéant de manière automatique ou semi-automatique, le procédé de production de métabolites selon l'invention.

Comme le montre ladite figure 8, cette installation est essentiellement constituée, d'une part, par une pluralité de contenants 2 pour la culture hors sol de plantes 1, renfermant ou non un support ou substrat de culture inerte 2", d'autre part, par un dispositif de stockage et d'apport de solution nutritive 3 pour les plantes 1, comprenant un réservoir 13 et une ligne de distribution 13' associée à un réseau de conduits 13" amenant ladite solution 3 de manière contrôlée à la base desdites plantes 1, et par un dispositif de distribution de solution de perméabilisation et/ou de liquide de lessivage, comprenant notamment un réservoir de mélange et de stockage 14 pour la solution de perméabilisation et/ou un réservoir 14' pour le liquide de lessivage, associé(s) à des lignes de distribution 14" par aspersion et/ou injection et, enfin, par un ensemble de moyens 15, 15', 15", 16 de récupération, de traitement et de recyclage au moins de la solution nutritive usagée 3'.

Les moyens de récupération, de traitement et de recyclage consistent avantageusement en un bac de stockage intermédiaire 15 collectant la solution nutritive usagée 3' évacuée par drainage des contenants 2 de culture hors sol et relié par une ligne de recyclage directe 15' et par une seconde ligne de recyclage 15" comprenant une unité 16 de séparation ou d'extraction des métabolites recherchés, en une boucle de réinjection sur le réservoir de stockage 13 de solution nutritive 3, ce dernier étant également alimenté par une ligne d'injection 13''' de substances nutritionnelles additionnelles stockées dans des réservoirs 13"" adaptés, de manière à compenser les quantités de telles substances prélevées par les plantes 1.

Pour augmenter la productivité de l'installation, cette dernière peut également comprendre un dispositif de distribution de solution(s) de précurseur(s) des métabolites à synthétiser par les plantes et/ou de substances stimulant ladite synthèse de métabolites, comprenant un réservoir de stockage 17 et une ligne de distribution 17' reliée simultanément à la ligne de distribution 13' ou au réseau de conduits 13", délivrant la solution nutritive 3 et à des moyens 18 de distribution par aspersion des plantes 1 au niveau de leurs parties aériennes, tout comme le dispositif 14, 14', 14" de distribution de solution de perméabilisation et/ou de liquide de lessivage.

Conformément à une caractéristique supplémentaire de l'invention, représentée également sur la figure 8, l'installation peut, en outre, comporter. d'une part, des moyens 19', 19" de chauffage des plantes 1 et/ou de leurs contenants 2, par exemple sous la forme de réseaux de conduits parallèles et espacés traversés par un fluide caloriporteur et s'étendant au-dessus et/ou en dessous desdits contenants 2 et, d'autre part, des moyens 20 d'éclairage ou d'insolation artificiels desdites plantes 1.

Le fonctionnement de l'installation représentée sur la figure 8 est décrit plus précisément ci-après.

La solution nutritive 3 est préparée et stockée dans un premier réservoir 13. Elle est ensuite amenée à la base des plantes 1 par un système de tuyauteries de plus en plus petites formant un réseau de conduits 13". Les plantes sont en culture hors sol, éventuellement sur un support inerte 2". La solution nutritive usagée 3' s'écoule sur un support de préférence en pente formant éventuellement le fond du contenant 2 correspondant, ce drainage 5 étant permanent, cyclique ou occasionnel. Ces eaux de drainages sont récupérées dans le bac de stockage 15. Elles sont ensuite recyclées directement ou bien les molécules qu'elles contiennent en sont extraites et purifiées par l'unité 16 avant recyclage. Un premier réservoir annexe facultatif 14, et le cas échéant un second réservoir annexe 14', contient ou contiennent des agents perméabilisants, des précurseurs ou d'autres composés stimulant la production de métabolites chez les végétaux. Ces produits, mélangés à la solution nutritive ou non, sont mis en contact avec les plantes 1 au niveau des racines par percolation (injection dans la solution nutritive 3) ou au niveau des feuilles par aspersion. Les plantes sont soumises à un éclairage naturel ou artificiel 20. Les parties aériennes et racinaires des plantes peuvent être réchauffées ou refroidies par des liquides caloriporteurs circulant dans des arrangements de tuyauteries formant des réseaux 19', 19" ou d'autres moyens équivalents.

En relation avec les différentes variantes de réalisation de mise en oeuvre de l'invention, et en particulier avec le mode de réalisation représenté à la figure 8, ledit procédé sera avantageusement constitué de trois étapes principales. à savoir, une première étape de culture ayant pour but l'obtention d'une biomasse importante, une seconde étape privilégiant la libération de métabolites par les plantes, soit au niveau de leurs parties racinaires, soit au niveau de leurs partie aériennes et une troisième étape visant à remettre en état lesdites plantes avant passage à nouveau à la première ou à la deuxième étape précitée (répétition de cycles).

La présente invention concerne par conséquent un procédé et un système permettant la récupération de métabolites libérés naturellement (pas de perméabilisation indispensable) ou non (perméabilisation indispensable) par la plante cultivée en conditions hors sol (définition de Morard 1995), avec ou sans support inerte (par culture hors sol, il faut comprendre tout mode de culture autre que la culture en pleine terre dans les champs ou dans la nature). Cette plante peut être cultivée par arrosage avec une solution nutritive adaptée [culture hors sol sur support poreux, sur cascade (méthode dite NFT selon Morard 1995)], par trempage permanent dans la solution nutritive avec bullage d'air (aquiculture selon Morard 1995) ou immersion temporaire dans ladite solution nutritive (subirrigation, hydroponie, film nutritif,...), ou par mise en contact avec ladite solution nutritive sous forme de brouillard (aéroponie, selon Morard 1995).

Si les métabolites sont libérés par les racines de la plante, ils seront contenus dans la solution nutritive (percolat) et donc récupérés par piégeage soit dans un solvant non miscible à l'eau, soit sur un support adsorbant lesdites molécules ou encore par évaporation ou sublimation de l'eau de la solution nutritive. Ces différents modes opératoires peuvent être combinés si nécessaire.

Si les métabolites sont libérés par les parties aériennes de la plante. avec ou sans perméabilisation, ils seront récupérés par douchage desdites plantes, la solution de douchage (lessivat) étant récupérée et les métabolites qu'elle contient extraits comme indiqué ci-dessus pour le percolat.

Dans tous les cas, l'eau, la solution nutritive usagée ou le liquide de lessivage chargé peut être réutilisé pour la culture des plantes après remise à niveau des éléments minéraux et organiques nécessaires à la croissance et au métabolisme secondaire de la plante. Si un traitement perméabilisant a été effectué, il peut être nécessaire de traiter correctement la solution usagée (percolat ou lessivat) avant sa réutilisation pour un nouveau traitement perméabilisant ou pour une réutilisation comme solution nutritive normale (pour la croissance des plantes).

Lorsque les métabolites ont été extraits de la solution nutritive par un solvant non miscible à l'eau, ce dernier est séparé de la phase aqueuse, évaporé et le résidu sec est récupéré. Les métabolites peuvent ensuite être purifiés et séparés d'éventuels contaminants. Si les métabolites ont été piégés sur un support solide, on isole ce support solide de la solution nutritive, on élue les métabolites avec une phase liquide aqueuse en jouant sur la force ionique, sur le pH ou en ajoutant des contre ions ou bien on utilise un solvant organique miscible à l'eau ou non (alcool, hydrocarbure, organochloré, nitrile, ou autre...). Dans ce dernier cas, on va privilégier les solvants les plus respectueux de l'environnement, les moins inflammables, les moins toxiques, les moins chers et les plus biodégradables. Dans tous les cas, on mettra en oeuvre tous les moyens classiques de la chromatographie et de la chimie pour purifier et concentrer les molécules contenues dans l'éluat.

Lorsque les plantes ont été perméabilisées pendant le temps défini comme optimal pour obtenir la sortie maximale de métabolites sans affection irréversible de leur survie (perméabilisation séquentielle forcée sur des périodes courtes ou perméabilisation plus douce sur des périodes plus longues), le traitement perméabilisant est stoppé. Les plantes sont remises en conditions de culture normale. Lorsqu'elles ont été traitées par une solution perméabilisante, une solution de rinçage est utilisée pour éliminer cet agent perméabilisant (tensioactif, détergent, sel, acide, base...). Cette solution remplace temporairement la solution nutritive. Ensuite seulement, les plantes sont remises en culture en conditions normales. Après une période de culture suffisante, les plantes peuvent à nouveau être perméabilisées. Ces cycles peuvent être réitérés jusqu'à diminution notable de la production, de la productivité, de l'état sanitaire des plantes. Dans ce cas, tout ou partie de la culture est remplacée par des jeunes plantes qui sont mises en croissance avant le premier traitement perméabilisant, préférentiellement jusqu'à obtention d'un développement optimal.

Dans certains cas, un traitement perméabilisant en continu peut être envisagé. Dans ce cas, le traitement perméabilisant sera assez doux pour permettre la sortie des métabolites et surtout la survie de la plante tout au long dudit traitement perméabilisant. Lorsque l'efficacité du traitement commence à chuter de manière significative, et engendre une baisse de rendement non économiquement acceptable, on remplace les « vieilles » plantes par de plus jeunes que l'on perméabilisera immédiatement ou après forçage de la croissance.

De manière générale, lors de la perméabilisation des plantes, l'ajout de précurseurs des métabolites recherchés peut être fait. Dans ce cas, ces molécules peuvent être biotransformées par les cellules de ladite plante. L'ajout de précurseurs doit se faire avec modération pour éviter que ces derniers ne servent de source de carbone à des micro-organismes dont le développement pourrait à terme compromettre la survie desdites plantes. Cet ajout de précurseurs peut se faire directement dans la solution nutritive lorsqu'on perméabilise les racines. Il peut se faire par la solution nutritive via les racines si on perméabilise les feuilles aussi, mais il peut également être fait par voie foliaire via le liquide de douchage des plantes.

Afin d'illustrer davantage encore les possibles mises en oeuvre pratiques du procédé selon l'invention, on décrit ci-après quatre exemples non limitatifs de réalisation de ce dernier.

Exemple 1 de réalisation du procédé : Production d'alcaloïdes tropaniques à partir de Datura innoxia Mill. cultivés en hydroponie

Les plantes utilisées proviennent de graines fournies par l'Institut für Pflanzengenetik und Kulturpflanzenforschung (D-06466 Gatersleben, Corrensstr.3, Allemagne). Ces graines ont été scarifiées dans l'acide sulfurique concentré pendant 10 minutes à température ambiante. Après rinçage abondant à l'eau courante, elles ont été mises à germer dans des pots contenant du sol humide et à une température de 25°C+/-1°C avec une photopériode de 16h et une hygrométrie de 60-70%. Au bout de deux mois, lorsque les plantes ont atteint une taille de 15-20cm, c'est-à-dire un stade 8-10 feuilles, elles ont été dépotées et transférées dans des pots de verre de type connu sous la désignation "baby food jar" (Sigma-Aldrich Inc., France) de 175 ml. Les plantes étaient maintenues par le couvercle des pots préalablement percés de trois trous (un pour la tige, un pour le bullage d'air et un pour la remise à niveau du liquide. Voir figure 1). Une solution nutritive de type MS diluée quatre fois (Murashige et Skoog, 1962) a été ajoutée dans le pot. Des tuyaux en silicone ont permis de faire buller de l'air dans la solution nutritive afin de maintenir une bonne oxygénation pour le système racinaire des plantes et aussi, pour éviter le développement trop important de populations de micro-organismes. Soixante plantes ont ainsi été mises en place. Elles ont été cultivées pendant deux semaines dans les mêmes conditions de lumière. de température et d'hygrométrie que celles indiquées ci-dessus.

Des doses croissantes (0, 1, 2, 3 ou 5%V/V - volume pour volume) de tensioactif (du type connu sous la désignation Tween20 : polyoxyéthylènesorbitan monolaurate) ont été ajoutées à la solution nutritive. La perméabilisation au Tween 20 (commercialisé par la société Prolabo) a été réalisée en l'absence de bullage d'air (pour éviter la formation de mousse) pendant 0, 12, 24 ou 48 heures. La solution nutritive de chaque plante a été récoltée, filtrée et analysée par chromatographie en phase gazeuse. La concentration totale moyenne en hyoscyamine et en scopolamine augmente graduellement avec la concentration en tensioactif. Elle passe de 7mg/l sans tensioactif à 45mg/l avec 5% de Tween20. La concentration en hyoscyamine et en scopolamine augmente avec la durée du traitement.

L'ensemble des solutions de perméabilisation a été récupéré et les molécules qu'elles contenaient ont été reprises dans du chloroforme après adjonction d'ammoniaque jusqu'à pH 9. Le chloroforme a été évaporé et le résidu sec contenait l'ensemble des deux molécules d'hyoscyamine et de scopolamine.

Les plantes perméabilisées ont subi un rinçage du système racinaire à l'eau courante et ont été remises en culture hydroponique comme indiqué ci dessus. Au bout de trois semaines de culture, toutes les plantes avaient survécu. Elles avaient poursuivi leur croissance et certaines parmi celles qui avaient été perméabilisées ont même eu une croissance plus forte que les plantes témoins.

Un mois plus tard, les plantes ayant été perméabilisées une première fois l'ont été une seconde fois selon plusieurs modalités :
a) 4 plantes témoins (non perméabilisées)
b) 4 plantes perméabilisées pendant 24heures avec 3% d'un tensioactif connu sous la désignation de Teepol (marque déposée - société TEMANA)
c) 4 plantes perméabilisées pendant 24heures avec 5% de Tween20
d) 4 plantes perméabilisées pendant 24heures avec 5% de Tween20 placées sous éclairement permanent à 27°C
e) 4 plantes perméabilisées pendant 24heures avec 5% de Tween20 + 1g/l d'ornithine et 1g/l de phénylalanine
les solutions nutritives ont été prélevées, filtrées et analysées comme indiqué ci-dessus.

Les concentrations en hyoscyamine et en scopolamine étaient les suivantes :
a) 1,5mg/l+/-1
b) 2,4mg/l+/-1,4
c) 9,1mg/l+/-4
d) 18,8mg/l+/-3,7
e) 22,7mg/l+/-2,4

Conclusion : Il est possible de faire pousser des Daturas en aquiculture. Les Daturas libèrent spontanément des alcaloïdes (hyoscyamine et scopolamine) dans la solution nutritive. On peut forcer cette libération (également appelée par la suite relargage) en perméabilisant les plantes avec des tensioactifs. Dans ce cas, il est possible d'avoir un effet perméabilisant tout en évitant d'affecter la survie desdites plantes en jouant sur la nature du tensioactif, sa concentration et le temps de contact. Le Tween 20 est un tensioactif efficace pour cette application. A dose égale, le Tween 20 est plus efficace que le Teepol. L'ajout de précurseurs de l'hyoscyamine et de la scopolamine est très bénéfique en terme de quantité d'alcaloïdes libérés dans la solution nutritive.. Le passage des plantes à un éclairement permanent pendant 24h et à une température plus élevée donne des résultats proches de ceux obtenus avec ajout de précurseurs. Dans ce cas, on peut penser qu'une optimisation du système pourrait permettre d'augmenter encore largement la productivité du procédé.

Les plantes perméabilisées ont été rincées et remises en culture. Toutes ont survécu et ont donné lieu à des perméabilisations ultérieures.

Il résulte de ce qui précède que les plantes de type Datura peuvent être cultivées en conditions hors sol pour leur faire produire des alcaloïdes tropaniques que l'on récupère dans la solution nutritive usagée. La production peut être augmentée en procédant à un traitement adapté. Ce traitement peut se faire à l'aide de tensioactifs. Tous les tensioactifs ne sont pas aussi efficaces les uns que les autres. Plus la dose de tensioactif est élevée, plus le traitement est efficace. Plus le traitement est long plus il est efficace. Un traitement réalisé dans de bonnes conditions permet la survie de la plante, voire un développement supplémentaire de celle-ci, et il est alors possible de la perméabiliser plusieurs fois. L'efficacité du traitement perméabilisant peut être renforcée par ajout de précurseurs dans la solution perméabilisante. Cette efficacité peut aussi être renforcée par forçage de la photosynthèse en soumettant les plantes à un éclairement permanent, à une augmentation du taux de CO₂ et/ou par élévation de la température environnante.

Exemple 2 de réalisation du procédé : Production de taxanes à partir d'ifs en culture hors sol.

Quatre plantes d'ifs de 60cm de hauteur ont été achetés dans le commerce, dépotés et placés dans des pots en PVC (polychlorure de vinyle) d'une contenance de 6 litres et contenant 5 litres d'une solution nutritive de type MS (Murashige et Skoog, 1962)diluée 12 fois. Au bout de 4 jours de culture en serre avec bullage d'air (0,25vvm - 0,25 volume de gaz par volume de milieu et par minute) (Figure 2), la solution nutritive a été prélevée, filtrée sur filtres du type Millex 0,45µm et analysée par HPLC (chromatographie liquide haute pression) sur colonne C18 de 30 cm avec un gradient de 0 à 100% de méthanol et une détection UV à 254nm. Il n'a pas pu être détecté de taxol dans cette solution nutritive. La solution a été remplacée par une solution neuve et 2% de Tween20 ont été ajoutés dans le pot de culture de deux des plantes. Après 24 heures, une concentration de 2,1mg/l+/-1,04 (soit 5µg/l/g de matière fraîche) a été mesurée dans la solution nutritive des plantes perméabilisées alors que seulement 0,2mg/l+/-0,16 (soit 0,2µg/l/g de matière fraîche) ont pu être mesurés dans le cas des plantes non perméabilisées. Des résultats assez similaires sont obtenus pour le dosage de la baccatine 3 (autre métabolite secondaire intéressant chez l'if). Après rinçage des racines, les plantes ont pu être perméabilisées à nouveau sans compromettre leur viabilité pendant au moins les trois semaines de l'expérimentation. Ces résultats montrent donc qu'il est possible d'obtenir une libération partielle des taxanes (paclitaxel) à partir d'ifs en culture hors sol (dans ce cas, culture hydroponique de type aquiculture). Dans notre exemple, ni la solution nutritive, ni le traitement perméabilisant n'ont été optimisés. On peut donc s'attendre à des résultats plus intéressants encore après optimisation de ladite solution nutritive (voir Morard 1995), des conditions de culture (température, lumière, CO2...) et dudit traitement perméabilisant.

Exemple 3 de réalisation du procédé : Production de furocoumarines à partir de Rues (Ruta graveolens) cultivées en hydroponie (aquiculture selon Morard 1995).

Huit plantes âgées de 10 mois et cultivées en pot ont été placées en culture en solution nutritive MS/4 (solution de Murashige et Scoog 1962, diluée 4 fois) avec bullage d'air pendant deux semaines (même dispositif que sur la figure 2). Quatre des huit plantes ont été perméabilisées pendant 24 heures avec 3% (V/V) de Tween 20. Les solutions nutritives ont été ensuite prélevées, filtrées et analysées par chromatographie en phase gazeuse. Les solutions nutritives des plantes perméabilisées contenaient en moyenne 8,1+/-4,1mg/l de furocoumarines (psoralène, 8 méthoxypsoralène, 5 méthoxypsoralène et 5.8 diméthoxypsoralène) contre 5,2+/-2,8mg/l pour les solutions nutritives des quatre plantes témoins. Les racines des plantes ont été rincées à l'eau puis les plantes ont été remises en culture pour une semaine. Ensuite, les quatre plantes ayant déjà été perméabilisées la première fois ont été à nouveau perméabilisées de la même manière. Les résultats sont les suivants : 22,5+/-6,1mg/l pour les perméabilisées contre 19+/-8.5mg/l pour les non perméabilisées.

Une troisième perméabilisation a été refaite sur les mêmes plantes et a donné des résultats similaires avec survie de la totalité des plantes.

Conclusion : Les Rues peuvent être cultivées en conditions hors sol. Elles relarguent naturellement des furocoumarines dans la solution nutritive mais cette libération peut être augmentée par un traitement perméabilisant adapté (ici, le Tween 20 à 3%). D'autres types de traitements peuvent être envisagés de la même manière : autres tensioactifs, élévation de la température de la solution nutritive ou augmentation de la salinité de la solution nutritive, ajout d'umbelliferone ou de phénylalanine par exemple. Comme dans l'exemple 2, les conditions de culture et le traitement perméabilisant peuvent bien entendu être optimisés pour donner des rendements encore meilleurs.

Exemple 4 de réalisation du procédé : Culture de Datura innoxia et Datura stramonium en aéroponie

Des graines de Datura stramonium et de Datura innoxia ont été mises à germer dans des petits godets contenant de la vermiculite arrosée de solution nutritive. Ces godets ont ensuite été transférés dans des supports composés de plaques (de PVC) percées. Ces plaques ont été placées sur un grand bac de 1.2m X lm X lm au fond duquel six brumisateurs trempaient dans de la solution nutritive (figure 3). Les brumisateurs se déclenchaient pendant 15 minutes toutes les demi-heures. L'ensemble du dispositif a été placé en phytotron avec des conditions identiques à celles de l'exemple 1. Au bout d'un mois de culture, de quelques dizaines de grammes de matière fraîche, la biomasse totale a dépassé le kilogramme (tiges + feuilles + racines). Une analyse par HPLC a révélé la présence de 8mg/l d'alcaloïdes dans les 40 litres de solution nutritive, soit un total calculé de 320mg d'hyoscyamine et de scopolamine.

Conclusion : la croissance des Daturas en aéroponie est très rapide. Les plantes sont saines et vigoureuses. En outre, elles relarguent spontanément des alcaloïdes dans la solution nutritive. Ce relargage peut être amélioré par un traitement adapté tel que la perméabilisation, un changement de la solution nutritive avec augmentation de la concentration en sels ou augmentation de la température d'incubation des racines et éventuellement l'ajout de précurseurs des alcaloïdes tropaniques (Gontier 1993).

Il résulte de ce qui précède qu'on peut cultiver des Daturas en aéroponie et dans ce cas, la croissance du matériel végétal est très rapide. De grandes quantités de biomasse peuvent être obtenues en un temps plus court qu'en hydroponie (aquiculture selon Morard 1995). De plus, il est possible de récupérer des métabolites secondaires dans la solution nutritive usagée. La teneur en métabolites de cette solution est fonction de la quantité de biomasse et du temps de contact de la solution nutritive avec la partie racinaire de cette biomasse. On peut aussi imaginer d'augmenter la concentration en métabolites par un traitement adapté (perméabilisation, force ionique, température, élicitation).

Ainsi, dès lors qu'il est possible de faire pousser des plantes sur plusieurs milliers de mètres carrés de serre (c'est le cas pour la tomate et le concombre) et dès lors qu'il est possible de recycler les solutions nutritives usagées (c'est le cas en méthode de type aquiculture, aéroponique, NFT (Nutrient Film Technique), le procédé de l'invention peut permettre de cultiver sur plusieurs milliers de mètres carrés des plantes produisant des métabolites secondaires. On peut récupérer les métabolites relargués spontanément dans la solution nutritive usagée. On peut même forcer la libération de ces molécules ainsi que leur biosynthèse avant relargage. Chez les plantes qui produisent des métabolites secondaires qui sont ensuite "extrudés" ou "excrétés" à la surface des feuilles (Brown et Zobel 1990), on peut même récupérer ces composés par douchage desdites plantes avec une solution adaptée et récupérer les lessivats contenant les métabolites d'intérêt. Un tel dispositif servira à produire des molécules d'origine végétale et ces molécules pourront avoir diverses applications telles que thérapeutiques, cosmétiques, alimentaires ou non alimentaires. De préférence, ces molécules seront de masse moléculaire assez réduite et ne devront pas être totalement insolubles dans l'eau additionnée ou non de tensioactif.

Bien entendu, l'invention n'est pas limitée au mode de réalisation décrit et représenté aux dessins annexés. Des modifications restent possibles, notamment du point de vue de la constitution des divers éléments ou par substitution d'équivalents techniques, sans sortir pour autant du domaine de protection de l'invention, tel que défini par les revendications.

## Revendications

1. Procédé de production de molécules à partir de plantes, **caractérisé en ce que** lesdites plantes sont cultivées en conditions hors sol et sont alimentées par une solution nutritive et/ou aspergées par un liquide de lessivage, ladite solution et/ou ledit liquide étant ensuite récupéré(e)(s) et traité(e)(s) pour en extraire certaines molécules déterminées qu'elle ou il contient et qui ont été libérées par les racines et/ou les parties aériennes desdites plantes.

2. Procédé selon la revendication 1, **caractérisé en ce qu'**il consiste plus précisément à réaliser les étapes suivantes :
a) initiation de la culture des plantes en conditions hors sol permettant la croissance et le développement desdites plantes et l'obtention d'une biomasse suffisamment importante pour rentabiliser le procédé ;
b) récupération des métabolites libérés par les racines ou les parties aériennes des plantes au moyen d'un liquide mis en contact par percolation ou immersion avec les racines, ou par aspersion avec les feuilles desdites plantes ;
c) collecte de la solution usagée ou chargée sous forme de percolat (pour les racines) ou de lessivat (pour les feuilles) et isolement des molécules recherchées contenues dans cette solution par extraction liquide-liquide, liquide-solide, par recristallisation, par sublimation ou par évaporation de l'eau.

3. Procédé selon la revendication 2, **caractérisé en ce qu'**il comprend une étape supplémentaire de perméabilisation forcée des racines ou des parties aériennes des plantes avec une solution contenant notamment des sels, des tensioactifs, des détergents, des solvants, des éliciteurs d'origine fongique ou bactérienne, des dérivés de l'acide jasmonique ou d'autres produits stimulant les défenses naturelles des plantes, de telle sorte que les flux sortants des racines ou des feuilles vers la solution nutritive ou le liquide de lessivage soient augmentés de manière significative et ce, sans perte totale de la viabilité desdites plantes, afin que ces dernières puissent être réutilisées, directement ou après une phase de rinçage et de remise en conditions de culture permettant au moins le rétablissement d'un bon état physiologique desdites plantes.

4. Procédé selon la revendication 3, **caractérisé en ce que** la perméabilisation forcée est réalisée de manière permanente, en additionnant une solution perméabilisante à la solution nutritive délivrée de manière continue ou à intervalles réguliers aux plantes.

5. Procédé selon la revendication 3 ou la revendication 4, **caractérisé en ce que** l'on réalise un rinçage du système racinaire et/ou des parties aériennes des plantes, puis une remise en conditions de croissance desdites plantes pendant un temps juste suffisant pour permettre de réaliser à nouveau un traitement de perméabilisation forcée, suivi ou accompagné d'une étape de récupération des molécules recherchées.

6. Procédé selon l'une quelconque des revendications 1 à 5, **caractérisé en ce qu'**il consiste à renforcer les capacités de biosynthèse de la plante et son potentiel à relarguer des métabolites dans la solution, par l'intermédiaire d'un traitement physique choisi dans le groupe formé par une élévation de la température, une irradiation lumineuse, supplémentaire ou rallongée, une élévation de la teneur en CO₂ dans l'atmosphère en contact avec ladite plante et les combinaisons de plusieurs de ces traitements physiques.

7. Procédé selon l'une quelconque des revendications 1 à 6, **caractérisé en ce que** la production et l'excrétion des métabolites synthétisés par les plantes sont renforcées par une néosynthèse de ces molécules suite à l'apport de précurseurs de ces métabolites, ces précurseurs de la voie de biosynthèse desdits métabolites étant apportés à la plante par la solution nutritive au niveau des racines ou par aspersion sur les parties aériennes en mélange avec des tensioactifs pour obtenir une pénétration foliaire.

8. Procédé selon l'une quelconque des revendications 1 à 7, **caractérisé en ce que** la production et l'excrétion des métabolites sont renforcées par un forçage de l'activité photosynthétique au moyen d'un éclairement artificiel pendant une durée plus longue que celle à laquelle la plante est habituée.

9. Procédé selon l'une quelconque des revendications 1 à 8, **caractérisé en ce que** les métabolites recherchés sont obtenus à partir des parties aériennes des plantes par douchage ou aspersion desdites plantes avec une solution ou un liquide adapté(e), contenant préférentiellement des tensioactifs, des agents de surfaces et/ou des détergents, la solution de douchage ou d'aspersion chargée étant récupérée séparément ou en mélange avec la solution nutritive pour une extraction et une purification des molécules recherchées.

10. Procédé selon l'une quelconque des revendications 1 à 9, **caractérisé en ce que** les plantes utilisées sont choisies dans le groupe des plantes supérieures ou vascularisées, aptes à synthétiser des métabolites d'intérêt économique en grande quantité et **en ce que** les molécules recherchées correspondent à des métabolites secondaires produits naturellement par ces plantes.

11. Procédé selon l'une quelconque des revendications 1 à 10, **caractérisé en ce que** les métabolites récupérés et isolés sont choisis dans le groupe formé par les alcaloïdes de types tropaniques et indoliques, les anticancéreux, les taxanes ou dérivés du taxol, les furocoumarines, les terpènes, les glycosides, les phénylpropanoïdes, les saponines et les stéroïdes.

12. Procédé selon l'une quelconque des revendications 1 à 11, **caractérisé en ce que** les plantes sont modifiées génétiquement pour leur faire produire plus de métabolites ou pour modifier leur morphologie, en augmentant par exemple leur biomasse racinaire suite à une transformation génétique par Agrobacterium rhizogenes.

13. Installation pour la mise en oeuvre, notamment à grande échelle, du procédé de production selon l'une quelconque des revendications 1 à 12, **caractérisée en ce qu'**elle est essentiellement constituée, d'une part, par une pluralité de contenants (2) pour la culture hors sol de plantes (1), renfermant ou non un support ou substrat de culture inerte (2"), d'autre part, par un dispositif de stockage et d'apport de solution nutritive (3) pour les plantes (1), comprenant un réservoir (13) et une ligne de distribution (13') associée à un réseau de conduits (13") amenant ladite solution (3) de manière contrôlée à la base desdites plantes (1), et par un dispositif de distribution de solution de perméabilisation et/ou de liquide de lessivage, comprenant notamment un réservoir de mélange et de stockage (14) pour la solution de perméabilisation et/ou un réservoir (14') pour le liquide de lessivage, associé(s) à des lignes de distribution (14") par aspersion et/ou injection et, enfin, par un ensemble de moyens (15, 15', 15", 16) de récupération, de traitement et de recyclage au moins de la solution nutritive usagée (3'), ces moyens consistant en un bac de stockage intermédiaire (15) collectant la solution nutritive usagée (3') évacuée par drainage des contenants (2) de culture hors sol et relié par une ligne de recyclage directe (15') et par une seconde ligne de recyclage (15") comprenant une unité (16) de séparation ou d'extraction, en une boucle de réinjection sur le réservoir de stockage (13) de solution nutritive (3), ladite unité (16) extrayant les métabolites recherchés contenus dans la solution nutritive usagée (3') et libérés par les racines et/ou les parties aériennes desdites plantes (1).

14. Installation selon la revendication 13, **caractérisée en ce que** le réservoir (13) est également alimenté par une ligne d'injection (13"') de substances nutritionnelles additionnelles stockées dans des réservoirs (13"") adaptés, de manière à compenser les quantités de telles substances prélevées par les plantes (1).

15. Installation selon l'une quelconque des revendications 13 et 14, **caractérisée en ce qu'**elle comprend également un dispositif de distribution de solution(s) de précurseur(s) des métabolites à synthétiser par les plantes et/ou de substances stimulant ladite synthèse de métabolites, comprenant un réservoir de stockage (17) et une ligne de distribution (17') reliée simultanément à la ligne de distribution (13') ou au réseau de conduits (13"), délivrant la solution nutritive (3) et à des moyens (18) de distribution par aspersion des plantes (1) au niveau de leurs parties aériennes, tout comme le dispositif (14, 14', 14") de distribution de solution de perméabilisation et/ou de liquide de lessivage.

16. Installation selon l'une quelconque des revendications 13 à 15, **caractérisée en ce qu'**elle comporte également, d'une part, des moyens (19', 19") de chauffage des plantes (1) et/ou de leurs contenants (2), par exemple sous la forme de réseaux de conduits parallèles et espacés traversés par un fluide caloriporteur et s'étendant au dessus et/ou en dessous desdits contenants (2) et, d'autre part, des moyens (20) d'éclairage ou d'insolation artificiels desdites plantes (1).

## Claims

1. Method for producing molecules from plants, **characterised in that** the said plants are cultivated in soil-less conditions and are supplied with a nutrient solution and/or sprayed with a leaching liquid, the said solution and/or the said liquid being subsequently recovered and treated to extract therefrom certain specific molecules which it contains and which have been released by the roots and/or top growth of the said plants.

2. Method according to claim 1, **characterised in that** it consists more specifically in embodying the following stages:
a) initiation of the growing of the plants in soil-less conditions allowing the growth and development of the said plants and obtaining a biomass sufficiently significant to make the method profitable;
b) recovery of the metabolites released by the roots or top growth of the plants through a liquid which is put into contact with the roots by percolation or immersion, or with the leaves of the said plants by spraying;
c) collection of the used or charged solution as a percolate (for the roots) or as a leachate (for the leaves) and isolation of the sought molecules contained in this solution by liquid-liquid or liquid-solid extraction, by recrystallisation, by sublimation or by evaporation of the water.

3. Method according to claim 2, **characterised in that** it includes an additional stage of forced permeabilisation of the roots or top growth of the plants with a solution containing in particular salts, surfactants, detergents, solvents, elicitors of fungal or bacterial origin, derivatives of jasmonic acid or other products which stimulate the natural defences of the plants, such that the flux from the roots or leaves into the nutrient solution or the leaching liquid is significantly increased and this, without the total loss of the viability of the said plants, in order that the plants can be reused, either directly or after a rinsing phase and be returned to growing conditions allowing at least the reestablishment of a good physiological state of the said plants.

4. Method according to claim 3, **characterised in that** the forced permeabilisation is made in a permanent manner, by adding a permeabilising solution to the nutrient solution provided in a continuous manner or at regular intervals to the plants.

5. Method according to claim 3 or 4, **characterised in that** the root system and/or the top growth of the plants is rinsed, then put back into growth conditions of the said plants for a sufficient amount of time to allow treatment again for forced permeabilisation, followed or accompanied by a stage of recovering the sought molecules.

6. Method according to any of claims 1 to 5, **characterised in that** it aims to reinforce the biosynthesis capacities of the plant and its potential to release the metabolites into the solution, by the intermediary of a physical treatment chosen from the group including raising the temperature, an additional or extended light radiation, raising the level of CO₂ in the atmosphere in contact with the said plant and combinations of several of these physical treatments.

7. Method according to any of claims 1 to 6, **characterised in that** the production and excretion of the metabolites synthesised by the plants are reinforced by a neosynthesis of these molecules following the provision of precursors of these metabolites, these precursors of the biosynthesis process of the said metabolites being supplied to the plant through the nutrient solution at root level or by spraying the top growth mixed with the surfactants to obtain penetration of the leaves.

8. Method according to any of claims 1 to 7, **characterised in that** the production and excretion of the metabolites are reinforced by forcing the photosynthetic activity by means of artificial lighting for a longer period than that to which the plant is accustomed.

9. Method according to any of claims 1 to 8, **characterised in that** the sought metabolites are obtained from the top growth of the plants by dampening or spraying the said plants with an adapted solution or liquid, preferably containing the surfactants, surface agents and/or detergents, the charged dampening or spraying solution being recovered separately or mixed with the nutrient solution for an extraction or purification of the sought molecules.

10. Method according to any of claims 1 to 9, **characterised in that** the plants used are chosen from the group of superior or vascular plants, which are capable of synthesising economically viable large quantities of metabolites and in which the sought molecules correspond to the secondary metabolites which are produced naturally by these plants.

11. Method according to any of claims 1 to 10, **characterised in that** the metabolites which are recovered and isolated are chosen from the group formed by tropane and indole alkaloids, the anti-canceroids, the taxanes or derivatives of taxol, furocoumarins, terpenes, glycosides, phenylpropanoids, saponins and steroids.

12. Method according to any of claims 1 to 11, **characterised in that** the plants are genetically modified to make them produce more metabolites or to modify their morphology, by increasing, for example, their root biomass following a genetic transformation by Agrobacterium rhizogenes.

13. System for implementing, notably on a grand scale, the production method according to any of claims 1 to 12, **characterised in that** it essentially comprises, firstly, a plurality of containers (2) for the soil-less cultivation of plants (1), which may or may not contain a support or substrate of inert culture (2"), secondly, a device for stocking and supplying a nutrient solution (3) for the plants (1), comprising a reservoir (13) and a distribution line (13') linked to a network of conduits (13") bringing the said solution (3) in a controlled manner to the base of the said plants (1), and a device for distributing the permeabilisation solution and/or leaching liquid, comprising notably a mixing and stocking reservoir (14) for the permeabilisation solution and/or a reservoir (14') for the leaching liquid, linked to the distribution lines (14") by spraying and/or injection, and finally an assembly of means (15, 15', 15", 16) for the recovery, treatment and recycling at least of the used nutrient solution (3'), these means consisting of an intermediate stocking vat (15) which collects the used nutrient solution (3') which is evacuated by draining the containers (2) of soil-less culture and linked by a direct recycling line (15') and by a second recycling line (15") comprising a separation or extraction unit (16), in a re-injection loop on the stocking reservoir (13) of the nutrient solution (3), the said unit (16) extracting the sought metabolites contained in the used nutrient solution (3') and released by the roots and/or the top growth of the said plants (1).

14. System according to claim 13, **characterised in that** the reservoir (13) is also fed by an injection line (13"') with additional nutritional substances stocked in suitable reservoirs (13''''), in a manner to compensate the quantities of such substances already removed by the plants (1).

15. System according to any of claims 13 and 14, **characterised in that** it also comprises a device for the distribution of precursor solution(s) of the metabolites to be synthesised by the plants and/or substances stimulating the said synthesis of metabolites, comprising a stocking reservoir (17) and a distribution line (17') linked simultaneously to the distribution line (13') or to the network of conduits (13"), providing the nutrient solution (3) and to the means (18) for distribution by spraying the plants (1) at the level of the top growth, all like the device (14, 14', 14") for distributing the permeabilisation solution and/or leaching liquid.

16. System according to any of claims 13 to 15, **characterised in that** it also comprises, firstly, means (19', 19") of heating the plants (1) and/or their containers (2), for example in the form of networks of parallel conduits spaced out and crossed by a heat-carrying fluid and extending above and/or under the said containers (2) and, secondly, the means (20) for artificially lighting or insulating the said plants (1).

## Patentansprüche

1. Verfahren zur Herstellung von Molekülen aus Pflanzen, **dadurch gekennzeichnet, daß** die Pflanzen unter erdlosen Bedingungen kultiviert und mit einer Nährlösung versorgt und/oder mit einer waschflüssigkeit besprüht werden, wobei die Lösung und/oder die Flüssigkeit anschließend wiedergewonnen und behandelt wird, um daraus einige bestimmte, darin enthaltene Moleküle zu extrahieren, die von den Wurzeln und/oder den oberirdischen Teilen der Pflanzen abgegeben werden.

2. Verfahren nach Anspruch 1, **dadurch gekennzeichnet, daß** es im einzelnen in der Durchführung der folgenden Schritte besteht:
a) Initiierung der Pflanzenkultur unter erdlosen Bedingungen, so daß die Pflanzen wachsen und sich entwickeln können und Biomasse in ausreichender Menge erhalten werden kann, um das Verfahren rentabel zu machen;
b) Gewinnung der Metaboliten, die von den Wurzeln oder den oberirdischen Teilen der Pflanze abgegeben werden, mit Hilfe einer Flüssigkeit, die durch Perkolieren oder Eintauchen mit den Wurzeln oder durch Besprühen mit den Blättern der Pflanzen in Kontakt gebracht wird;
c) Auffangen der gebrauchten bzw. beladenen Lösung in Form eines Perkolats (bei den Wurzeln) oder einer Auswaschflüssigkeit (bei den Blättern) und Isolierung der gesuchten, in dieser Lösung enthaltenen Moleküle durch Flüssig/flüssig-Extraktion, Flüssig/fest-Extraktion, Umkristallisieren, Sublimieren oder durch Verdampfen des Wassers.

3. Verfahren nach Anspruch 2, **dadurch gekennzeichnet, daß** es einen zusätzlichen Schritt der erzwungenen Permeabilisierung der Wurzeln oder der oberirdischen Teile der Pflanzen mit einer Lösung umfaßt, die insbesondere Salze, Tenside, Detergentien, Lösungsmittel, Elicitoren aus Pilzen oder Bakterien, Derivate der Jasmonsäure oder andere Substanzen enthält, die die natürliche Abwehr der Pflanzen in einer Weise stimulieren, daß sich der Stoffstrom aus den wurzeln oder Blättern in die Nährlösung bzw. in die Waschflüssigkeit deutlich erhöht, ohne daß die Lebensfähigkeit der Pflanzen völlig verlorengeht, so daß sie direkt wiederverwendet werden können oder nach einer Phase des Spülens und Rücksetzens in Kulturbedingungen, die zumindest die Wiederherstellung eines guten physiologischen Zustandes der Pflanzen ermöglichen.

4. Verfahren nach Anspruch 3, **dadurch gekennzeichnet, daß** die erzwungene Permeabilisierung permanent erfolgt, indem eine permeabilisierende Lösung zur Nährlösung gegeben wird, die den Pflanzen kontinuierlich oder in regelmäßigen Abständen zugeführt wird.

5. Verfahren nach Anspruch 3 oder 4, **dadurch gekennzeichnet, daß** eine Spülung des Wurzelsystems und/oder der oberirdischen Teile der Pflanzen durchgeführt wird, anschließend Rücksetzen in Pflanzenwachstumsbedingungen über einen hinreichenden Zeitraum erfolgt, um die Durchführung einer erneuten Behandlung mit erzwungener Permeabilisierung zu ermöglichen, gefolgt oder begleitet von einem Schritt der Gewinnung der gesuchten Moleküle.

6. Verfahren nach einem der Ansprüche 1 bis 5, **dadurch gekennzeichnet, daß** es darin besteht, die Biosynthesekapazität der Pflanze und ihr Potential zur Ausscheidung von Metaboliten in die Lösung durch eine physikalische Behandlung zu verstärken, die ausgewählt ist aus der Gruppe bestehend aus Erhöhung der Temperatur, ergänzender oder längerer Bestrahlung mit Licht, Erhöhung des CO₂-Gehalts in der mit der Pflanze in Kontakt stehenden Atmosphäre sowie Kombinationen aus mehreren dieser physikalischen Behandlungen.

7. Verfahren nach einem der Ansprüche 1 bis 6, **dadurch gekennzeichnet, daß** Produktion und Ausscheidung der von den Pflanzen synthetisierten Metaboliten durch Neusynthese dieser Moleküle nach Bereitstellung von Vorläufern dieser Metaboliten verstärkt werden, wobei diese Vorläufer bei der Biosynthese der Metaboliten der Pflanze mit der Nährlösung an den Wurzeln zugeführt werden oder durch Besprühen der oberirdischen Teile in Mischung mit Tensiden, um Eindringen in die Blätter zu erreichen.

8. Verfahren nach einem der Ansprüche 1. bis 7, **dadurch gekennzeichnet, daß** Produktion und Ausscheidung der Metaboliten verstärkt werden durch Steigern der Photosyntheseaktivität mittels künstlicher Beleuchtung über eine Zeitdauer, die länger ist als die, an die die Pflanze gewöhnt ist.

9. Verfahren nach einem der Ansprüche 1 bis 8, **dadurch gekennzeichnet, daß** die gesuchten Metaboliten aus den oberirdischen Teilen der Pflanzen durch Bespritzen oder Besprühen der Pflanzen mit einer geeigneten Lösung oder Flüssigkeit erhalten werden, die vorzugsweise Tenside, oberflächenaktive Mittel und/oder Detergentien enthält, wobei die beladene Spritz- oder Sprühlösung für sich oder in Mischung mit der Nährlösung zur Extraktion und Reinigung der gesuchten Moleküle wiedergewonnen wird.

10. Verfahren nach einem der Ansprüche 1 bis 9, **dadurch gekennzeichnet, daß** die verwendeten Pflanzen ausgewählt sind aus der Gruppe der höheren Pflanzen oder Gefäßpflanzen, die in der Lage sind, wirtschaftlich interessante Metaboliten in großer Menge zu synthetisieren, und daß die gesuchten Moleküle sekundären Metaboliten entsprechen, die natürlicherweise von diesen Pflanzen produziert werden.

11. Verfahren nach einem der Ansprüche 1 bis 10, **dadurch gekennzeichnet, daß** die gewonnenen und isolierten Metaboliten ausgewählt sind aus der Gruppe bestehend aus Alkaloiden vom Tropan- und Indol-Typ, gegen Krebs wirksamen Verbindungen, Taxanen oder Taxol-Derivaten, Furocumarinen, Terpenen, Glycosiden, Phenylpropan-Verbindungen, Saponinen und Steroiden.

12. Verfahren nach einem der Ansprüche 1 bis 11, **dadurch gekennzeichnet, daß** die Pflanzen genetisch verändert sind, um sie mehr Metaboliten produzieren zu lassen oder ihre Morphologie zu verändern, beispielsweise durch Erhöhen ihrer Wurzelbiomasse nach genetischer Transformation mit *Agrobacterium rhizogenes*.

13. Anlage zur Durchführung, insbesondere in großem Maßstab, des Herstellungsverfahrens nach einem der Ansprüche 1 bis 12, **dadurch gekennzeichnet, daß** die Anlage im wesentlichen aufgebaut ist aus einerseits mehreren Behältern (2) für die erdlose Kultur der Pflanzen (1), enthaltend einen inerten Kulturträger bzw. ein solches Substrat (2") oder nicht, und zum anderen einer Vorrichtung zur Lagerung und Bereitstellung von Nährlösung (3) für die Pflanzen (1), umfassend einen Tank (13) und eine Versorgungsleitung (13'), die mit einem Leitungsnetz (13") verbunden ist, das die Lösung (3) kontrolliert zur Unterlage der Pflanzen (1) bringt, sowie einer Vorrichtung zur Abgabe der Permeabilisierungslösung und/oder der Waschflüssigkeit, umfassend insbesondere einen Misch- und Vorratstank (14) für die Permeabilisierungslösung und/oder einen Tank (14') für die Waschflüssigkeit, der/die mit Versorgungsleitungen (14") zum Besprühen und/oder Einspritzen verbunden ist/sind, und schließlich einer Gruppe aus Einrichtungen (15, 15', 15", 16) zur Wiedergewinnung, Behandlung und Rückführung zumindest der gebrauchten Nährlösung (3'), wobei diese Einrichtungen einen Bottich zur Zwischenlagerung (15) umfassen, in dem die gebrauchte Nährlösung (3') aufgefangen wird, die durch Entleerung der Behälter (2) für die erdlose Kultur abgeführt wird, und der mit einer direkten Rückführungsleitung (15') und einer zweiten Rückführungsleitung (15"), umfassend eine Trennoder Extrahiereinheit (16), zu einer Reinjektionsschleife zum Vorratstank (13) für die Nährlösung (3) verbunden ist, wobei die Einheit (16) die gesuchten Metaboliten extrahiert, die in der gebrauchten Nährlösung (3') enthalten sind und von den Wurzeln und/oder den oberirdischen Teilen der Pflanzen (1) abgegeben wurden.

14. Anlage nach Anspruch 13, **dadurch gekennzeichnet, daß** der Tank (13) über eine Einspritzleitung (13"') auch mit zusätzlichen Nährstoffen versorgt wird, die in geeigneten Tanks (13"") gelagert werden, um so die Menge derjenigen Substanzen auszugleichen, die von den Pflanzen (1) aufgenommen wurden.

15. Anlage nach einem der Ansprüche 13 und 14, **dadurch gekennzeichnet, daß** die Anlage auch eine Vorrichtung zur Abgabe von Lösung(en) des/der Vorläufer(s) der von den Pflanzen zu synthetisierenden Metaboliten und/oder von Substanzen umfaßt, die die Synthese der Metaboliten stimulieren, umfassend einen Vorratstank (17) und eine Versorgungsleitung (17), die gleichzeitig mit der Versorgungsleitung (13') bzw. mit dem Leitungsnetz (13") verbunden ist, durch die die Versorgung mit Nährlösung (3) erfolgt, sowie mit Einrichtungen (18) zur Abgabe durch Besprühen der Pflanzen (1) an ihren oberirdischen Teilen, sowie die Vorrichtung (14, 14', 14") zur Abgabe von Permeabilisierungslösung und/oder Waschflüssigkeit.

16. Anlage nach einem der Ansprüche 13 bis 15, **dadurch gekennzeichnet, daß** die Anlage zum einen auch Einrichtungen (19', 19") zur Beheizung der Pflanzen (1) und/oder ihrer Behälter (2) umfaßt, beispielsweise in Form eines Netzes paralleler und beabstandeter Leitungen, durch die eine Wärmetauscherflüssigkeit strömt und die oberhalb und/oder unterhalb der Behälter (2) verlaufen, sowie zum anderen Einrichtungen (20) zur künstlichen Beleuchtung oder Bestrahlung der Pflanzen (1).
